# EUROPÄISCHE PATENTSCHRIFT

(11) **EP 0 729 450 B1**
(45) Veröffentlichungstag und Bekanntmachung des Hinweises auf die Patenterteilung: **22.04.1998**
(21) Anmeldenummer: 95901371.5
(22) Anmeldetag: 11.11.1994
(51) Int. Cl.: C07C 217/50, C11D 1/835, C07C 213/08, C07C 219/06, C07C 219/08

(54) **VERFAHREN ZUR HERSTELLUNG VON FESTEN ESTERQUATS**
METHOD FOR PREPARATION OF SOLID QUATERNARY ESTERS
PROCEDE DE PREPARATION D'ESTERS QUATERNAIRES SOLIDES

(30) Priorität: 20.11.1993 DE 4339643
(43) Veröffentlichungstag der Anmeldung: 04.09.1996
(73) Patentinhaber: Henkel Kommanditgesellschaft auf Aktien, 40191 Düsseldorf (DE)
(72) Erfinder: WAHLE, Bernd, D-41564 Kaarst (DE); BIGORRA LLOSAS, Joaquim, E-08203 Sabadell (ES); PI, Rafael, E-08400 Granollers (ES); SOLER CODINA, Antoni, E-08221 Terassa (ES); BRAU BALAGUE, Emili, E-43570 Santa-Barbara (ES); JANSEN, Yvonne, D-47829 Krefeld (DE); WALTENBERGER, Peter, D-53547 Breitscheid (DE)
(86) Internationale Anmeldenummer: EP9403743
(87) Internationale Veröffentlichungsnummer: WO9514654

(56) Entgegenhaltungen:
- EP-A- 0 008 839
- EP-A- 0 284 036
- EP-A- 0 569 847
- EP-A- 0 604 726

## Beschreibung

Die Erfindung betrifft ein Verfahren zur Herstellung fester Esterquats, bei dem man Fettsäuretriethanolaminester in Gegenwart von ausgewählten Dispergatoren quaterniert sowie die Verwendung der genannten Esterquats zur Herstellung von oberflächenaktiven Mitteln.

### Stand der Technik

Für die Behandlung von Textilfasern, -garnen oder -geweben wird eine Vielzahl von Verbindungen oder Stoffgemischen vorgeschlagen, die den damit behandelten Textilien erwünschte Eigenschaften verleihen oder die Bestandteile von Mitteln zur Textilpflege sind. Je nach Art der angewendeten Wirkstoffe können dabei die Verarbeitungseigenschaften, die Trageeigenschaften der Textilien wie auch deren Pflege verbessert werden.

In den letzten Jahre haben auf dem Gebiet der Textilbehandlungsmittel Produkte an Bedeutung gewonnen, die als kationische Tensidkomponente im wesentlichen quaternierte Estersalze von Fettsäuren mit Alkanolaminen, sogenannte "Esterquats", enthalten. Stellvertretend für den umfangreichen Stand der Technik sei an dieser Stelle auf die Druckschriften **US 3,915,867, US 4,370,272, EP 0 239 910 A2, EP 0 293 955 A2, EP 0 295 739 A2** und **EP 0 309 052 A2** verwiesen.

Esterquats zeichnen sich nicht nur durch ausgezeichnete Avivage- und Antistatikeigenschaften aus, sondern weisen zudem eine für kationische Tenside überraschend gute biologische Abbaubarkeit auf. Von Nachteil ist jedoch, daß Esterquats üblicherweise in Form von wäßrigen Dispersionen in den Handel gelangen, die nicht immer eine zufriedenstellende Lagerstabilität aufweisen. So beoabchtet man häufig eine nachteilige Änderung der Viskosität, Phasentrennung oder Sedimentation. Weiter unerwünscht ist der Umstand, daß die Esterquats üblicherweise alkoholische Konzentrate darstellen, die mit Wasser auf die Anwendungskonzentration verdünnt werden müssen. Auch hier besteht ein Bedürfnis nach Produkten, die lösungsmittelfrei sind.

Die Aufgabe der Erfindung hat somit darin bestanden, ein Verfahren zur lösungsmittelfreien Herstellung von festen Esterquats zu entwickeln, die stabile wäßrige Dispersionen einer gleichbleibend vorteilhaften Viskosität bilden.

### Beschreibung der Erfindung

Gegenstand der Erfindung ist ein Verfahren zur Herstellung fester Esterquats, bei dem man Fettsäuretriethanolaminester der Formel **(I)**, 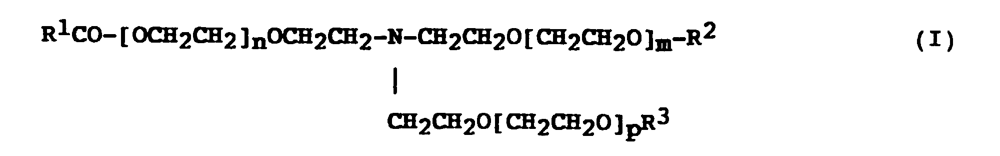 in der R¹CO für einen gesättigten und/oder ungesättigten Acylrest mit 6 bis 22 Kohlenstoffatomen, R² und R³ unabhängig voneinander für Wasserstoff oder R¹CO und n, m und p in Summe für 0 oder Zahlen von 1 bis 10 steht, in Gegenwart von
a) Fettalkoholpolyglycolethern und
b) Fettsäurepartialglyceriden
in an sich bekannter Weise mit Alkylierungsmitteln quaterniert.

Überraschenderweise wurde gefunden, daß sich die Quaternierung von Fettsäuretriethanolaminestern auch in Gegenwart der genannten Dispergatoren durchführen läßt. Auf diese Weise werden lösemittelfreie, insbesondere alkoholfreie, feste Esterquats erhalten, die in zweifacher Hinsicht stabile Dispersionen bilden: zum einen findet auch bei längerer Lagerung weder eine Phasentrennung noch eine Sedimentation statt, zum anderen weisen die Dispersionen eine konstante Viskosität auf. Die Erfindung schließt die Erkenntnis ein, daß der nachträgliche Zusatz der genannten Dispergatoren zu nach konventionellen Verfahren hergestellten Esterquats das Dispergiervermögen nicht oder nur sehr geringfügig verbessert.

### Esterquats und Fettsäuretriethanolaminester

Esterquats stellen eine bekannte Gruppe kationischer Tenside dar, die üblicherweise durch Veresterung von Triethanolamin bzw. Triethanolaminpolyglycolethern mit Fettsäuren und nachfolgende Quaternierung in organischen Lösungsmitteln erhalten werden. Herstellung und Eigenschaften der Esterquats sind beispielsweise in der **WO 91/01 295** (Henkel) sowie den Übersichtsartikeln von O.Ponsati in **C.R. CED-Kongress, Barcelona, 167 (1992)** und R.Puchta in **C.R. CED-Kongress, Sitges, 59 (1993)** beschrieben.

Im Sinne des erfindungsgemäßen Verfahrens kommen als Ausgangsstoffe für die Herstellung der Esterquats Fettsäuretriethanolaminester der Formel **(I)** in Betracht, die vorzugsweise technische Mono-/Di-/Triester-Gemische darstellen, bei denen der Veresterungsgrad im Bereich von 1,2 bis 2,5, insbesondere 1,5 bis 1,9 liegt. Besonders bevorzugt sind Ester, die sich von technischen C_{12/18}- bzw. C_{16/18}-Fettsäuren, wie beispielsweise Palmfettsäure, Kokosfettsäure oder Talgfettsäure ableiten und eine Iodzahl im Bereich zwischen 0 und 40 aufweisen können.

### Dispergatoren

Als erste Dispergatorkomponente kommen Fettalkoholpolyglycolether der Formel **(II)** in Betracht,

**R**^{**4**}**O-(CH**_{**2**}**CH**_{**2**}**O)**_{**q**}**H (II)**

in der R⁴ für einen linearen oder verzweigten aliphatischen Kohlenwasserstoffrest mit 6 bis 22 Kohlenstoffatomen und 0 und/oder 1, 2 oder 3 Doppelbindungen und q für Zahlen von 10 bis 50 steht.

Bei diesen Stoffen handelt es sich um bekannte nichtionische Tenside, die großtechnisch durch basenkatalysierte Anlagerung von Ethylenoxid an Fettalkohole hergestellt werden. Typische Beispiele sind Addukte von 10 bis 50, vorzugsweise 25 bis 45 und insbesondere 30 bis 40 Mol Ethylenoxid an Capronalkohol, Caprylalkohol, 2-Ethylhexalalkohol, Caprinalkohol, Laurylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol und Erucylalkohol sowie deren technische Gemische, die beispielsweise durch Hochdruckhydrierung von nativen Fettsäuremethylesterfraktionen oder Aldehyden aus der Roelen' schen Oxosynthese erhalten werden können. Vorzugsweise werden Anlagerungsprodukte von durchschnittlich 30 bis 40 Mol Ethylenoxid an technische C_{12/18}-Kokos- oder C_{16/18}-Talgalkoholschnitte eingesetzt.

Als zweite Dispergatorkomponente kommen Fettsäurepartialglyceride in Betracht, bei denen es sich vorzugsweise um technische Gemische von Mono- und/oder Diestern des Glycerins mit Fettsäuren der Formel **(III)** handelt,

**R**^{**5**}**CO-OH (III)**

in der R⁵CO für einen aliphatischen Acylrest mit 6 bis 22 Kohlenstoffatomen steht. Typische Beispiele sind Mono- und/ oder Diglyceridgemische auf Basis von Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technischen Gemischen, wie sie beispielsweise bei der Druckspaltung von Fetten und Ölen anfallen. Vorzugsweise werden Monoglyceride von Laurinsäure, Palmitinsäure, Stearinsäure, Ölsäure sowie C_{12/18}-Kokosfettsäure und C_{16/18}-Talgfettsäure eingesetzt.

Wäßrige Dispersionen mit besonders vorteilhafter Stabilität werden erhalten, wenn man feste Esterquats verwendet, zu deren Herstellung man die Fettsäuretriethanolaminester, die Fettalkoholpolyglycolether und die Fettsäurepartialglyceride im Gewichtsverhältnis (40 bis 60) : (10 bis 25) : (15 bis 50) und vorzugsweise (44,5 bis 51,0) : (16,0 bis 18,0) : (32,5 : 39,0) eingesetzt hat, mit der Maßgabe, daß sich die Angaben zu 100 Gewichtsteilen addieren.

### Alkylierung

Die Alkylierung der Fettsäuretriethanolaminester kann in an sich bekannter Weise durchgeführt werden. Hierzu wird der Ester zusammen mit der Dispergatormischung vorgelegt und mit dem Alkylierungsmittel - das man üblicherweise in äquimolaren Mengen oder leichtem Unterschuß einsetzt - bei erhöhten Temperaturen gerührt. Nach Abschluß der Reaktion kann nichtumgesetztes Alkylierungsmittel durch Zugabe einer geringen Menge Aminosäure, vorzugsweise Glycin, zerstört werden. Als Alkylierungsmittel kommen in diesem Zusammenhang Alkylhalogenide, Dialkylsulfate und Ethylenoxid - letzteres in Gegenwart von Dialkylphosphaten - in Betracht. Vorzugsweise betrifft das erfindungsgemäße Verfahren methylquaternierte Esterquats in Form ihrer Chloride oder Methylsulfat-Salze sowie Esterquat-Salze, die mit 1 bis 5 Mol Ethylenoxid quaterniert worden sind.

### Gewerbliche Anwendbarkeit

Die nach dem erfindungsgemäßen Verfahren erhältlichen Esterquats lassen sich leicht in Wasser dispergieren. Die resultierenden Dispersionen weisen auch bei längerer Lagerung eine konstante Viskosität auf und zeigen keine Tendenz zur Phasentrennung oder Sedimentation. Materialien, die mit den nach dem erfindungsgemäßen Verfahren hergestellten Esterquats behandelt worden sind, zeichnen sich durch ein erhöhtes Wiederbenetzungsvermögen aus.

Ein weiterer Gegenstand der Erfindung betrifft daher die Verwendung der nach dem erfindungsgemäßen Verfahren erhältlichen festen Esterquats zur Herstellung von oberflächenaktiven Mitteln, insbesondere Wäscheweichspül- und Avivage- und Haarpflegemitteln, in denen sie in Mengen von 10 bis 100, vorzugsweise 20 bis 60 Gew.-% - bezogen auf den Feststoffgehalt der Mittel - enthalten sein können. Unter Haarpflegemittel sind in diesem Zusammenhang beispielsweise Haarshampoos, Haarspülungen, Haarfestiger, Fönfestiger und dergleichen zu verstehen; vorzugsweise weisen die Mittel einen pH-Wert im Bereich von 3 bis 5, vorzugsweise 3 bis 4 auf.

Die folgenden Beispiele sollen den Gegenstand der Erfindung näher erläutern, ohne ihn darauf einzuschränken.

### Beispiele

### I. Herstellung der Esterquats

### Beispiel 1:

a) **Veresterung**. In einem 1-l-Dreihalskolben mit Rührer, Innenthermometer und Destillationsaufsatz wurden 324 g (1,2 mol) teilgehärtete C_{16/18}-Talgfettsäure (Iodzahl = 40), 149 g (1 mol) Triethanolamin und 1,4 g 50 gew.-%ige unterphosphorige Säure gegeben. Über einen Zeitraum von 4 h wurde die Reaktionsmischung bei einem verminderten Druck von 40 mbar auf eine Temperatur von 160°C erhitzt, bis die Säurezahl unterhalb von 5 lag. Anschließend wurde der rohe Talgfettsäuretriethanolaminester abgekühlt, der Reaktionsansatz entspannt und unter ständigem Rühren innerhalb von 15 min 1 Liter Luft durchgeleitet.
b) **Quaternierung**. In einem 500-ml-Dreihalskolben mit Rührer, Tropftrichter und Rückflußkühler wurde eine Mischung bestehend aus
   b1) 69,2 g (0,153 mol), entsprechend 44,5 Gew.-% des Esters aus la),
   b2) 25,6 g (0,012 mol), entsprechend 16,5 Gew.-% C_{16/18}-Talgalkohol-40EO und
   b3) 60,7 g (0,169 mol), entsprechend 39,0 Gew.-% Glycerinmonostearat (Monomuls^{(R)} 90S18, CF Grünau, Illertissen/(FRG)
   vorgelegt und unter Rühren auf 45°C erhitzt.

Innerhalb von 2 h wurden 18,9 g (0,15 mol) Dimethylsulfat zugetropft. Nach Beendigung der Zugabe wurde die Mischung weitere 2 h bei 60°C gerührt und nichtumgesetztes DMS durch Zusatz von 0,4 g (0,005 mol) Glycin zerstört. Die wasserfreien Esterquat/Emulgator-Gemische wurden als hellfarbige, wachsartige Massen erhalten, die gegebenenfalls anschließend mechanisch geschuppt wurden.

### Beispiel 2:

Beispiel 1 wurde wiederholt, die Quaternierung jedoch mit einer Mischung der folgenden Zusammensetzung durchgeführt:
b1) 89,3 g (0,197 mol), entsprechend 57,5 Gew.-% des Esters aus a),
b2) 15,5 g (0,007 mol), entsprechend 10,0 Gew.-% C_{16/18}-Talgalkohol-40EO und
b3) 50,4 g (0,141 mol), entsprechend 32,5 Gew.-% Glycerinmonostearat.

### Beispiel 3:

Beispiel 1 wurde wiederholt, die Quaternierung jedoch mit einer Mischung der folgenden Zusammensetzung durchgeführt:
b1) 73,4 g (0,159 mol), entsprechend 46,7 Gew.-% des Esters aus a),
b2) 49,3 g (0,021 mol), entsprechend 31,7 Gew.-% C_{16/18}-Talgalkohol-40EO und
b3) 33,4 g (0,09 mol), entsprechend 21,6 Gew.-% Glycerinmonostearat.

### II. Anwendunastechnische Beispiele

Jeweils 10 g der wasserfreien, festen Esterquats aus I) wurden in 90 g Wasser gelöst und der pH-Wert der Mischungen auf 3,3 eingestellt. Die Bildung der Dispersionen erfolgte unter schwachem Rühren bei Raumtemperatur. In allen Fällen wurden homogene Dispersionen erhalten. Die Viskosität der Dispersionen wurde nach 1, 2 und 15 d Lagerung bestimmt (Brookfield RVT, Spindel 2, 25°C, 20 Upm), die Stabilität nach 15 d optisch beurteilt. Die Ergebnisse sind in Tab.1 zusammengefaßt:

**Tab.1:**

| Viskositätsmessungen | | | | | |
|---|---|---|---|---|---|
| Bsp. | Herstellbeispiel | Viskosität (mPas) | | | Stabilität |
| | | 1 d | 2 d | 15 d | 15 d |
| 4 | 1 | 100 | 98 | 98 | +++ |
| 5 | 2 | 100 | 98 | 98 | +++ |
| 6 | 3 | 100 | 98 | 98 | +++ |
| +++) = keine Phasentrennung, keine Sedimentation | | | | | |

### Vergleichsversuche

Zum Vergleich wurde ein handelsübliches Esterquat (Dehyquart^{(R)} AU 46, 90 Gew.-%ig in Isopropylalkohol, Fa.Pulcra S.A., Barcelona/ES) zunächst vom Lösungsmittel befreit, anschließend - also nachträglich - mit den genannten Dispergatoren in den angegebenen Mengenverhältnissen vermischt. In allen Vergleichsversuchen zeigte sich, daß zur Dispersionsbildung eine deutlich höhere Scherleistung erforderlich war. Im Hinblick auf die Viskosität der Vergleichsdispersionen wurden zwar ähnliche Anfangswerte erreicht, jedoch schon nach kurzer Lagerung eine rasche Viskositätsabnahme beobachtet. Ferner zeigten die Vergleichsdispersionen eine geringere Lagerstabilität.

## Patentansprüche

1. Verfahren zur Herstellung fester Esterquats, bei dem man Fettsäuretriethanolaminester der Formel **(I)**, 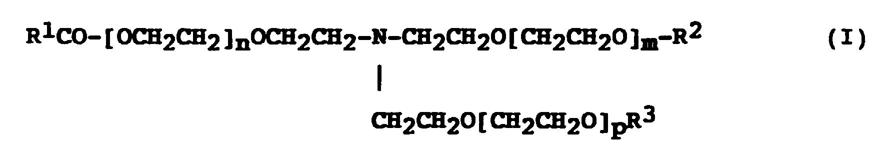 in der R¹CO für einen gesättigten und/oder ungesättigten Acylrest mit 6 bis 22 Kohlenstoffatomen, R² und R³ unabhängig voneinander für Wasserstoff oder R¹CO und n, m und p in Summe für 0 oder Zahlen von 1 bis 10 steht, in Gegenwart von
a) Fettalkoholpolyglycolethern und
b) Fettsäurepartialglyceriden
in an sich bekannter Weise mit Alkylierungsmitteln quaterniert.

2. Verfahren nach Anspruch 1, **dadurch gekennzeichnet**, daß man Fettalkoholpolyglycolether der Formel **(II)** einsetzt,
**R**^{**4**}**O-(CH**_{**2**}**CH**_{**2**}**O)**_{**q**}**H (II)**
in der R⁴ für einen linearen oder verzweigten aliphatischen Kohlenwasserstoffrest mit 6 bis 22 Kohlenstoffatomen und 0 und/oder 1, 2 oder 3 Doppelbindungen und q für Zahlen von 10 bis 50 steht.

3. Verfahren nach den Ansprüchen 1 und 2, **dadurch gekennzeichnet**, daß man als Fettsäurepartialglyceride technische Gemische von Mono- und/oder Diestern des Glycerins mit Fettsäuren der Formel **(III)** einsetzt,
**R**^{**5**}**CO-OH (III)**
in der R⁵CO für einen aliphatischen Acylrest mit 6 bis 22 Kohlenstoffatomen steht.

4. Verfahren nach den Ansprüchen 1 bis 3, **dadurch gekennzeichnet**, daß man die Fettsäuretriethanolaminester, die Fettalkoholpolyglycolether und die Fettsäurepartialglyceride im Gewichtsverhältnis (40 bis 60) : (10 bis 25) : (15 bis 50) einsetzt, mit der Maßgabe, daß sich die Angaben zu 100 Gewichtsteilen addieren.

5. Verfahren nach den Ansprüchen 1 bis 4, **dadurch gekennzeichnet**, daß man als Alkylierungsmittel Alkylhalogenide, Dialkylsulfate oder Ethylenoxid einsetzt.

6. Verwendung von festen Esterquats hergestellt nach dem Verfahren nach den Ansprüchen 1 bis 5 zur Herstellung von oberflächenaktiven Mitteln.

## Claims

1. A process for the production of solid esterquats, in which fatty acid triethanolamine esters corresponding to formula **(I)**: 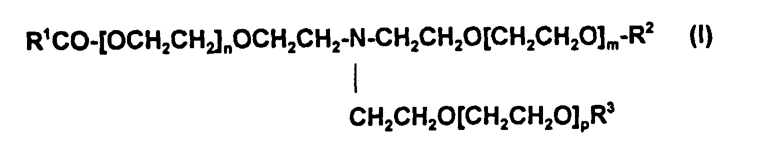 in which R¹CO is a saturated and/or unsaturated acyl radical containing 6 to 22 carbon atoms, R² and R³ independently of one another represent hydrogen or have the same meaning as R¹CO and n, m and p together stand for 0 or for numbers of 1 to 10,
are quaternized in known manner with alkylating agents in the presence of
a) fatty alcohol polyglycol ethers and
b) fatty acid partial glycerides.

2. A process as claimed in claim 1, characterized in that fatty alcohol polyglycol ethers corresponding to formula **(II)**:
**R**^{**4**}**O-(CH**_{**2**}**CH**_{**2**}**O)**_{**q**}**H (II)**
in which R⁴ is a linear or branched aliphatic hydrocarbon radical containing 6 to 22 carbon atoms and 0 and/or 1, 2 or 3 double bonds and q is a number of 10 to 50,
are used.

3. A process as claimed in claims 1 and 2, characterized in that technical mixtures of monoesters and/or diesters of glycerol with fatty acids corresponding to formula **(III)**:
**R**^{**5**}**CO-OH (III)**
in which R⁵CO is an aliphatic acyl radical containing 6 to 22 carbon atoms, are used as the fatty acid partial glycerides.

4. A process as claimed in claims 1 to 3, characterized in that the fatty acid triethanolamine esters, the fatty alcohol polyglycol ethers and the fatty acid partial glycerides are used in a ratio by weight of (40 to 60) : (10 to 25) : (15 to 50), with the proviso that the figures add up to 100 parts by weight.

5. A process as claimed in claims 1 to 4, characterized in that alkyl halides, dialkyl sulfates or ethylene oxide are used as the alkylating agents.

6. The use of the solid esterquats produced by the process claimed in claims 1 to 5 for the production of surface-active formulations.

## Revendications

1. Procédé de fabrication d'esters quaternaires solides, dans lequel on quaternise de façon connue avec des agents alkylants des esters de triéthanolamine d'acides gras de Formule (I), 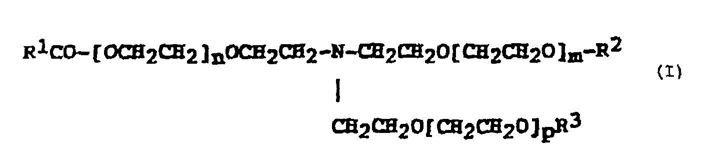 dans laquelle R¹CO représente un radical acyle saturé et/ou insaturé ayant de 6 à 22 atomes de carbone, R² et R³ représentent indépendamment l'un de l'autre un hydrogène ou R¹CO, et n, m et p valent au total 0, ou des nombres allant de 1 à 10, en présence de
a) polyglycoléthers d'alcools gras, et
b) glycérides partiels d'acides gras.

2. Procédé selon la revendication 1,
caractérisé en ce qu'
on utilise des polyglycoléthers d'alcools gras de Formule (II)
R⁴O - (CH₂CH₂O)_{q}H (II)
dans laquelle R⁴ représente un radical hydrocarboné aliphatique linéaire ou ramifié, ayant de 6 à 22 atomes de carbone, et 0 et/ou 1, 2 ou 3 doubles liaisons, et q représente un nombre allant de 10 à 50.

3. Procédé selon les revendications 1 et 2,
caractérisé en ce qu'
on utilise comme glycérides partiels d'acides gras des mélanges industriels de mono- et/ou diesters de glycérine, avec des acides gras de formule (III)
R⁵CO-OH (III)
dans laquelle R⁵CO représente un radical acyle aliphatique ayant de 6 à 22 atomes de carbone.

4. Procédé selon les revendications 1 à 3,
caractérisé en ce qu'
on utilise les esters de triéthanolamine d'acides gras, les polyglycoléthers d'alcools gras, et les glycérides partiels d'acides gras dans un rapport pondéral de (40 à 60) : (10 à 25) : (15 à 50), sous réserve que les indications totalisent 100 parties en poids.

5. Procédé selon les revendications 1 à 4,
caractérisé en ce qu'
on utilise comme agent alkylant des halogénures d'alkyle, des sulfates de dialkyle, ou l'oxyde d'éthylène.

6. Utilisation d'esters quaternaires fabriqués selon le procédé des revendications 1 à 5, pour préparer des agents tensioactifs.
